# EUROPEAN PATENT SPECIFICATION

(11) **EP 2 032 712 B1**
(45) Date of publication and mention of the grant of the patent: **10.02.2010**
(21) Application number: 07796600.0
(22) Date of filing: 29.06.2007
(51) Int. Cl.: C12P 17/04

(54) **REGULATION OF ACID METABOLITE PRODUCTION**
REGULATION DER SÄUREMETABOLITPRODUKTION
RÉGULATION DE LA PRODUCTION DE MÉTABOLITES ACIDES

(30) Priority: 29.06.2006 US 818145 P; 25.09.2006 US 847344 P
(43) Date of publication of application: 11.03.2009
(73) Proprietor: IVAX PHARMACEUTICALS S.R.O., 747 70 Opava 9 (CZ)
(72) Inventor: POKLUDA, Zdenek, 747 23 Bolatice (CZ); SATKE, Josef, 747 05 Opava 5 (CZ); VALA, Vladimir, PSC 735 52 Bohumin (CZ); VALIK, Josef, 747 05 Opava 5 (CZ)
(74) Representative: Wong, Kit Yee
(86) International application number: PCT/US2007/015234
(87) International publication number: WO 2008/002665

(56) References cited:
- EP-A- 1 624 070
- WO-A-99/20785
- WO-A-02/100855
- WO-A-2004/044183
- WO-A-2005/082902
- DE-A1- 4 407 441
- GB-A- 1 593 208

## Description

The present application claims the benefit of the following United States Provisional Patent Application Nos.: 60/818,145, filed June 29, 2006 and 60/847,344, filed September 25, 2006.

### FIELD OF THE INVENTION

The present invention relates to a method of regulating mycophenolic acid production by *Penicillium* having accession number CCM 8364 by regulation of the level of oxygen saturation in the culture medium, and to the newly isolated strain of *Penicillium* having accession number CCM 8364.

### BACKGROUND OF THE INVENTION

Mycophenolic acid (referred to as MPA) is a metabolite produced by several species of *Penicillium* (Imperial Chemical Industries GB 1 158 387 (1967), Lilly & Co Elli GB 1 593 208 (1978)) or by mutant strains obtained from *Penicillium* (Ajinimoto KK US 4 452 891 (1981)). MPA has biological and pharmacological activities e.g. as an antimicrobial (Florey et al., 1946), an antitumor agent (Williams et al., J. Antibiot., 21, 463 (1968), and as an immunosuppressive agent (Mitsui, Suzuki, 1969). In therapy, mycophenolate mofetil, a 2-morpholinoethyl ester derivative of MPA is commonly used.

The chemical structure of MPA is:

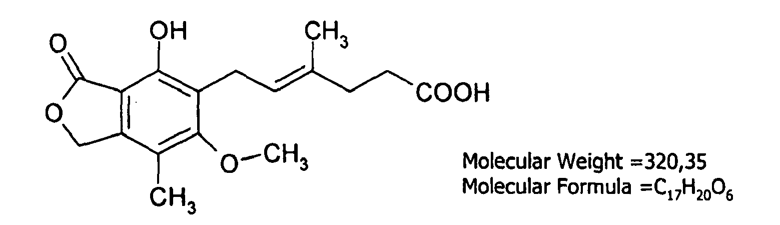

One of the methods to produce MPA is in a submerged culture fermentation, as described in EP 1 624 070 A1; wherein NaOH is used for regulating the pH of the fermentation medium, by keeping it between 4 and 7.

MPA may be isolated from the fermentation medium by a variety of processes including those disclosed in Imperial Chemical Industries GB 1158 387 (1967); BIOCON, WO 01/64931 A1 (2001); and IVAX, WO 2006/031665 (2006), WO 01/21607 and WO 2005/105768). The isolated MPA is then used for the production of mycophenolate mofetil (WO 02/100855).

EP 1624070 discloses a process for the production of MPA. DE 4407441 relates to processes for producing citric acid/citrate using yeast. WO 99/20785 is directed towards the production of glutamate using wild type Bacillus methanolicus.

A high level of MPA production is desirable for the efficient and economic production of drugs based on the active pharmaceutical ingredient mycophenolate mofetil. The present invention provides such a method.

### SUMMARY OF THE INVENTION

In one aspect, the present invention provides a method of regulating the production of an acid metabolite produced by a microbial cell, comprising regulating the oxygen saturation of a culture medium comprising the microbial cell, wherein the microbial cell is a single strain of *Penicillium* having the accession number of CCM 8364 and the acid metabolite is mycophenolic acid (MPA). In a preferred embodiment, the method comprises providing acid metabolite producing microbial cells in a submerged fermentation culture system and producing the acid metabolite from the microbial cells in such culture, wherein the oxygen saturation of the culture medium is regulated to control pH of the culture medium, wherein the microbial cell is a single strain of *Penicillium* having the accession number of CCM 8364 and the acid metabolite is mycophenolic acid (MPA).

In another aspect, the present invention provides a method of preparing mycophenolate mofetil comprising converting mycophenolic acid to mycophenolate mofetil, wherein the mycophenolic acid is prepared by regulating the oxygen saturation of a fermentation broth comprising a mycophenolic acid producing microbial cell, wherein the microbial cell is a single strain of *Penicillium* having the accession number of CCM 8364.

In yet another aspect, the present invention further provides a strain of *Penicillium sp*. deposited as accession number CCM 8364 at Czech Collection of Microorganisms (CCM) at Masaryk University, Bmo, Czech Republic. The strain of *Penicillium sp*. deposited as accession number CCM 8364 produces mycophenolic acid in high concentrations (in excess of lg MPA per liter of culture medium).

### BRIEF DESCRIPTION OF THE DRAWINGS

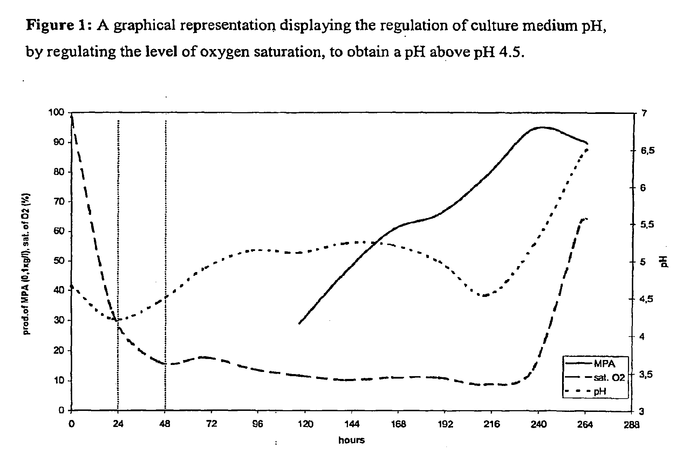

**Figure 1****:** A graphical representation displaying the regulation of culture medium pH, by regulating the level of oxygen saturation, to obtain a pH above pH 4.5.

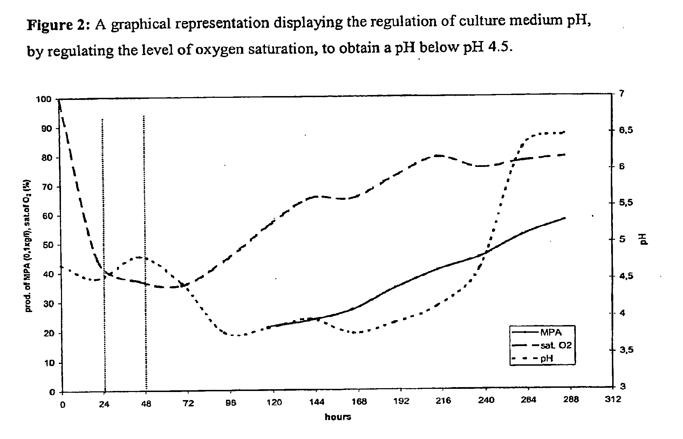

**Figure 2****:** A graphical representation displaying the regulation of culture medium pH, by regulating the level of oxygen saturation, to obtain a pH below pH 4.5.

### DETAILED DESCRIPTION OF THE INVENTION

As used herein, the term "high levels" or "high concentrations" when related to MPA refers to a level (or concentration) of more than about 1g of MPA per liter, preferably about 6 to about 10 g of MPA per 1 liter of fermentation broth (culture medium).

Several factors are important in obtaining high concentrations of MPA in the production medium of submerged culture fermentation. These factors include the composition of the medium and the cultivation conditions. In particular, an optimal pH of the fermentation broth has to be maintained during the production phase. Normally the addition of NaOH is used to regulate the pH of the fermentation broth and keep it between pH 4 and 7. NaOH is also used to produce a pH shift after the log phase of growth has ended. The present invention relates to a new method for regulating the production of MPA by regulating the pH of the fermentation broth/culture medium by regulating the level of oxygen without adding a chemical to the fermentation broth/culture medium. As such, the present invention uses only a natural source to regulate the pH of the fermentation broth/culture medium. Saturation by oxygen using air is performed by controlled aeration of this aerobic process, thereby regulating the pH of the process. Accordingly, the present process decreases consumption of hazardous acids and/or alkali compounds.

The present invention provides a method of regulating the production of MPA produced by Penicillium CCM 8364 by regulating the oxygen saturation of the fermentation broth. More specifically, the method comprises providing the microbial cell in a submerged fermentation culture system, wherein the oxygen saturation of the culture medium of the submerged fermentation culture is regulated/controlled, to produce an acid metabolite. Regulating or controlling the oxygen saturation of such culture medium ensures that the pH of the culture medium is maintained between pH 4 and pH 7, preferably between pH 4 and pH 6, more preferably between pH 4 and pH 5.5. Such regulation provides a method of producing the acid metabolite obtaining a high concentration of the acid metabolite in the fermentation broth (culture medium).

In the method of the present invention a suitable culture medium comprises a basic and a complex nitrogen source. The basic nitrogen source may be for example simple inorganic molecules, amino acids or other organic compounds containing nitrogen, such as for example ammonia/ammonium salts, any naturally occurring amino acid. A preferred complex nitrogen source is hydrolyzed casein and/or corn steep. The culture medium also contains a carbon source. A suitable carbon source for use in the culture medium may be a carbohydrate, preferably a sugar, more preferably a monosaccharide, most preferably sucrose.

In addition, the culture medium comprises a specific amino acid supplement in addition to the complex nitrogen source, or where the basic nitrogen source is a simple amino acid. Preferably, the amino acid supplement includes one or more of glycine, methionine and asparagine. Preferable methionine is D,L-methionine. Preferably asparagine is L-asparagine.

The amino acid in the amino acid supplement is commonly used at a concentration of about 1 to about 30g/l, preferably at a concentration of about 5 to about 8 g/l. Glycine is usually used at a concentration of about 1 to about 30 g/l, preferably glycine is used at a concentration of about 2 to about 20 g/l, more preferably glycine is used at a concentration of about 3 to about 15 g/l, even more preferably the concentration of glycine is about 5 to about 8 g/l.

D,L-methionine is commonly used at a concentration of about 0.01 to about 10 g/l more preferably D,L-methionine is used at a concentration of about 0.1 to about 1 g/l.

L-asparagine is commonly used at a concentration of about 1 to about 30 g/l, more preferably L-asparagine is used at a concentration of about 2 to about 20 g/l, yet more preferably L-asparagine is used at a concentration of about 3 to about 15 g/l, even more preferably the concentration of L-asparagine is about 5 to about 8 g/l.

The regulation of MPA production occurs when the *Penicillium* having the accession number of CCM 8364 is grown in a submerged fermentation culture having a defined oxygen saturation level. In such submerged fermentation culture the level of oxygen saturation in the culture medium is controlled during at least a part of the production phase. Alternatively the level of oxygen saturation is controlled throughout the entire production phase. As such the level of oxygen saturation in the culture medium may be controlled during the time period from 0 to 24 hours, or the time period from 0 to 48 hours, or from 24 to 48 hours or from 48 hours. Preferably, the level of oxygen saturation is controlled until the pH rises due to carbon source depletion at the end of the production phase.

The production of MPA in submerged fermentation may be increased by controlling the level of oxygen saturation in the culture medium. Preferably the level of oxygen saturation is in the range 5 to 40%, more preferably the level of oxygen saturation is in the range 5 to 35%, even more preferably the level of oxygen saturation is in the range from 5 to 30%. The % oxygen saturation refers to the pO₂ as measured by pO₂ electrode whereby 100% oxygen saturation is correlated to the maximal aeration of the culture medium without the microbial cell culture.

In a preferred embodiment, the *Penicillium* having the accession number of CCM 8364. is grown in a different culture medium in the inoculation and production stages of culture. The first stage (inoculation stage) starts at T=0 hours, a second stage may start at T=24 hours and the third stage (production stage) may start at T=48 hours.

Preferably, the process further comprises growing the microbial cells in an inoculating medium, and inoculating the production medium with an inoculum of at least 20%, preferably at least 25% of the volume of the production medium.

In the method of the present invention, the pH of the culture medium is maintained at a pH greater than pH 4, preferably between pH 4 and pH 7. Preferably, it is maintained by regulating the level of oxygen saturation in the culture medium. The level of oxygen saturation in the culture medium is regulated by continuous monitoring of the oxygen level using a pO₂ electrode and adjusting the oxygen level when necessary by adjusting the speed of agitation and added air volume. As such, the pH of the culture medium may be maintained at a pH greater than pH 4 during the production stage of the culture, which is the period at which time the acid metabolite is actively produced in the fermentation culture system. This period may start after an inoculate with the microbial cell has been transferred to the fermentation culture system. Preferably the pH is greater than pH 4.5 during the production stage of culture, more preferably the pH is between about pH 4.5 and about pH 5.5 during the production stage of culture.

The temperature of the submerged culture so regulated is maintained at about 20°C to about 30°C for a sufficient period of time to produce MPA. Preferably, the temperature of the submerged culture in the process of the present invention is maintained at about 24°C to about 26°C for a period of about 240 hours to about 300 hours.

The regulation of production of MPA according to the process of the present invention of regulating the level of oxygen saturation in the culture medium produces a final concentration of MPA (g/l of culture medium) that is in excess of 1 g/l, more preferably in excess of 2 g/l or in excess of 3 g/l, yet more preferably in excess of 4 g/l, even more preferably in excess of 5 g/l, in excess of 6g/l or in excess of 8 g/l.

The present invention also provides a new strain of *Penicillium sp.* (deposited as accession number CCM 8364) that produces high levels of MPA, such as for example in excess of 1g/l. This strain produces higher levels of MPA when compared to a wild type *Penicillium sp*. strain from which strain CCM 8364 was produced. Such wild type Penicillium sp. strain produces MPA with an average yield of about 600mg/l when grown in submerged culture.

Also provided is a culture of such strain, preferably a biologically pure culture, such as one comprising only growing cells of the fungal strain with deposit No CCM8364. Such culture has preferably at least about 90% of such fungal strain. This strain in culture may be in the form of individual cells, cell aggregates, hyphas, filaments, and its aggregates in or on any surface or environment.

The strain of *Pencillium sp.* having accession number CCM8364 is capable to produce a final concentration of MPA (g/l of culture medium) that is in excess of 1g/l, more preferably in excess of 2 g/l or in excess of 3 g/l, yet more preferably in excess of 4 g/l, even more preferably in excess of 5 g/l, in excess of 6g/l or in excess of 8 g/l.

The *Pencillium sp*. strain, CCM 8364, is produced by using a system of chemical and physical mutation methods combined with positive selection media.

The present invention further provides a process for preparing mycophenolate mofetil by preparing mycophenolic acid by the process of the present invention, and further converting it to mycophenolate mofetil. Mycophenolic acid may be converted to mycophenolate mofetil, for example, according to the process disclosed in WO 02/100855.

The following examples are intended to further illustrate certain preferred embodiments of the invention and are not limiting in nature.

### EXAMPLES

### Example 1: Growth Characteristics of Pencillium strain CCM 8364

### Macroscopic morphology

During saprophytic growth in agar-thickened malt medium - MEA - (35g malt extract, 5g peptone, 13g agar, 1000ml H₂O), strain CCM 8364 forms colonies of sphacelus airy mycelium having a diameter of 1.1cm after 7 days cultivation. The surface texture is velutinous, is centrally umbonate and has white margins. The level of conidiogenesis is moderate, the colonies are coloured near brown and have exudate present, buff, reverse buff.

During saprophytic growth in agar-thickened Czapek yeast medium, strain CCM 8364 forms colonies of sphacelus airy mycelium having a diameter of 2.3cm after 7 days cultivation. The surface texture is velutinous, is radially sulcate and centrally umbonate with white margins. The level of conidiogenesis is moderate, the colonies are coloured near brown and have exudate present, red brown, reverse cinnamon.

During saprophytic growth in agar-thickened production medium G25N, strain CCM 8364 forms colonies of sphacelus airy mycelium having a diameter of 0.3cm after 7 days cultivation. The surface texture is velutinous. The level of conidiogenesis is light to moderate, the colonies are centrally plane or are raised with white margins, coloured smoked grey - gloucous gray with greenish tinge, exudate absent, reverse pale.

### Microscopic morphology

The mycelium consist of stipes 300-600 x 4-6 µm, the surface is smooth, penicilli variable, terveticillate and irregular, with more branch points occurring in a non-uniform pattern, rami in verticils of 2-3, metulae in verticils, 12-20 x 4-4.5µm, phialides ampulliform - acerose, measuring 13-16 x 2.5-3µm, conidia in short chains, spheroidal 3.5-5.5µm and ellipsoidal 4-6 x 2-4µm with disjunctors, walls distinctly roughened.

### Example 2: MPA assay

Each isolate was evaluated for its MPA production. This assay consists of two steps - inoculation and production. The inoculation medium (IM) contains carbon sources (e.g. sucrose), nitrogen sources (suitable sources include hydrolyzed casein and corn steep) and inorganic salts (such as KH₂PO₄). 50ml of IM was sterilized at 120°C for 20 minutes in a 250ml Erlenmeyer flask. The spores of the isolate were washed off an agar slant culture with 20ml of sterile water. 5ml of this spore suspension was used as the inoculum for IM and then cultivated for 3-5 days at 24-28°C with a rate of stirring of 280 of revolutions/minute while shaking. The resulting IM was used for inoculation of the producing medium (PM) by the addition of 10% of the total volume.

The PM, utilized in the present invention for the assay of MPA, contains carbon sources (e.g. sucrose), nitrogen sources (suitable sources include casein and amino acids as glycine, asparagine, methionine) and inorganic salts (e.g. K₃PO₄, MgSO₄). 50 ml of the PM was sterilized at 120°C for 20 minutes in a 250ml Erlenmeyer flask. A test cultivation was then carried out under aerobic conditions at 24-28°C with shaking for 10 to 14 days. The rate of stirring was 280 revolutions/minute. The pH of the medium was measured before inoculation (pH 4.9 to pH 5.1) and two or three times before the end of the experiment using a pH meter as described above. The level of oxygen saturation was measured continuously in laboratory or plant fermenters. The MPA containing aliquots were assayed using HPLC chromatography according to standard techniques known to the person skilled in the art to determine their MPA content against a standard (internal IVAX standard MPA according Research report RR/RD/CH024/01-A, retest RR/RD/AN061/05-A). The column used was a Chromolith speed ROD, RP18e, 50x4.6mm, (manufactured by MERCK), mobile phase: 40% acetonitrile, 60% water containing 680µl H₃PO₄/liter H₂O, flow 5-6.5 ml/min and temperature 30°C.

### Example 3: Mutation and Selection Process

### 1. Selection

In the first stage of the procedure the sporulated slant of wild type *Penicillium sp*. was suspended in water and devoided of mycelia fragments. The suspension was then diluted to 100 spores/ml and spread on Petri plates containing MEA. The plates were then incubated at 24-27°C for seven days. The colonies which grew on the agar plate were picked-off and sustained on slants of the same nutrient agar medium in a Blacke flask. After 16 days of cultivation monocolony isolates were tested in the production test. The isolate with the best production (1.2g MPA/l in fermentation broth) was mutated in the next stage.

### 2. Mutating with N-methyl-N'-nitro-N-nitroso-guanidine (NG)

A sporulated slant was suspended in water and devoided of mycelia fragments. The conidia populations were then exposed to solution of 0.2M NG in saline for 8 hours. In this mutation treatment, approximately 99.9% of the spores were killed. The treated spores were then washed free of mutagen and spread on Petri plates containing MEA. The plates were incubated at 24-27°C for seven days. The colonies that grew on the agar plate were picked-off and sustained on slants of the same nutrient agar medium in a Blacke flask. After 16 days of cultivation monocolony isolates were tested in the production test. The isolate with the best production (2.4g MPA/l in fermentation broth) was mutated in the next stage.

### 3. Mutating with ethylmethanesulfonate (EMS)

A sporulated slant was suspended in water and devoided of mycelia fragments. The conidia populations were exposed to solution of 0.2M EMS in saline for 16 hours. In this mutation treatment approximately 99.9% of the spores were killed. The spores were then washed free of mutagen and inoculated on Petri plates containing MEA. The plates were incubated at 24-27°C for seven days until colonies formed on the plates. The colonies were picked-off and sustained on slants of the same nutrient agar medium in a Blacke flask. After 16 days cultivation monocolony isolates were tested in the production test. The isolate with the best production (5.6g MPA/l in fermentation broth) was mutated in the next stage.

### 4. Mutating with UV irradiation

A sporulated slant was suspended in water and devoided of mycelia fragments. The suspension was diluted to 1x10⁵ spores/ml and spread on Petri plates containing MEA. The plates were exposed to UV irradiation with the radiation dose being about 15 Jm⁻²s⁻¹ for about 500 seconds. In this mutation treatment approximately 99.9 % of the spores were killed. The Petri plates were then incubated at 24-27°C for seven days. The colonies that grew on the agar plate were picked-off and sustained on slants of the same nutrient agar medium in a Blacke flask. After 16 days of cultivation monocolony isolates were tested in the production test. The isolate with the best production (6.0g MPA/l in fermentation broth) was mutated in the next stage.

### 5. Mutating with Gamma irradiation

A sporulated slant was suspended in water and devoided of mycelia fragments. The suspension was diluted to 1x10⁵ spores/ml and exposed to Gamma radiation with radiation dose being 1500 Gy. Irradiated isolates were spread on Petri plates containing MEA. The Petri plates were then incubated at 24-27°C for seven days until colonies formed on the plates The colonies were picked-off and sustained on slants of the same nutrient agar medium in a Blacke flask. After 16 days of cultivation monocolony isolates were tested in the production test. The isolate with the best production (10g MPA/l in fermentation broth) was twice passaged on agar medium in a Blacke flask. After each passage the production test was performed to verify the continued high production of MPA.

This newly produced strain was deposited under the Budapest Treaty in the Czech Collection of Microorganisms (CCM) at the Masaryk University, Tvrdého 14, Bmo, Czech Republic under collection number CCM 8364.

The system of mutation procedures when combined with the positive selection procedures resulted in an increase in the average content of MPA from 600mg MPA/l of fermentation broth for the parent strain to 10g MPA/l of fermentation broth for strain CCM 8364 according to the MPA assay.

### Example 4: MPA production during fermentation in high oxygen saturation culture

Strain CCM 8364 was used for fermentation in a submerged fermenter with total volume about 14 cubic metres. The production medium contained sucrose as a carbon source and hydrolysed casein, glycine, asparagine and methionine as nitrogen sources and minerals including K, P and Mg at an initial pH of 5.0. The production medium was inoculated by a third stage inoculum (approximately 26 %w/w). The cultivation temperature was 24-26°C and the production fermentation run was 285 hours. The pH of cultivation medium was controlled by saturating it with oxygen to a level greater than 30%. The values of dissolved oxygen, pH during cultivation and the concentration of MPA at the end of production step are summarized in the following table. Due to oxygen saturation of the production media the pH dropped below pH 4 and the final MPA production level reached 5.7 g MPA/l.

| No. | Time (h) | 24 | 48 | 72 | 120 | 144 | 164 | 192 | 240 | MPA g/l |
|---|---|---|---|---|---|---|---|---|---|---|
| 1 | Oxygen saturation | 45% | 37% | 36% | 60% | 64% | 64% | 76% | 79% | 5.7 |
| | pH | 4.52 | 4.82 | 4.45 | 3.84 | 3.96 | 3.77 | 3.91 | 4.65 | |

### Example 5: MPA production during fermentation in medium oxygen saturation culture

Strain CCM 8364 was used for fermentation in a submerged fermenter with total volume about 14 cubic metres. The production medium contained sucrose as a carbon source and hydrolysed casein, glycine, asparagine and methionine as nitrogen sources and minerals including K, P and Mg at an initial pH of 5.0. The production strain *Penicillium sp.* (CCM 8364) was used for fermentation. The production medium was inoculated by the third stage inoculum (approximately 26 %, w/w). The cultivation temperature was 24-26°C and the production fermentation run was 278 hours. The pH of cultivation medium was controlled by means of saturation with oxygen. The values of dissolved oxygen, the pH of the culture medium during cultivation and the concentration of MPA at the end of production step are given in the following table.

| No. | Time (h) | 24 | 48 | 72 | 120 | 144 | 164 | 192 | 240 | MPA g/l |
|---|---|---|---|---|---|---|---|---|---|---|
| 2 | Oxygen saturation | 24% | 18% | 17% | 24% | 20% | 21% | 18% | 16% | 7.5 |
| | pH | 4.24 | 4.54 | 4.77 | 5.15 | 5.37 | 5.14 | 4.47 | 4.41 | |

### Example 6: MPA production during fermentation in low oxygen saturation culture

Strain CCM 8364 was used for fermentation in a submerged fermenter with total volume about 14 cubic metres. The production medium contained sucrose as a carbon source and hydrolysed casein, glycine, asparagine and methionine as nitrogen sources and minerals including K, P and Mg at an initial pH of 5.0. The production medium was inoculated by the third stage inoculum (approximately 26 %w/w). The cultivation temperature was 24-26°C. The fermentation broth was fed by medium with a carbon source (1x10% w/w) at 143 hours and the production fermentation was finished at 257 hours. The pH of cultivation medium was controlled by means of saturation with oxygen. Values for dissolved oxygen, pH of the culture medium during cultivation and concentration of MPA in the end of production step are given in the following table.

| No. | Time (h) | 24 | 48 | 72 | 120 | 144 | 164 | 192 | 240 | MPA g/l |
|---|---|---|---|---|---|---|---|---|---|---|
| 3 | Oxygen Saturation | 30% | 25% | 17% | 18% | 13% | 11% | 9% | 9% | 8.2 |
| | pH | 4.03 | 4.19 | 4.65 | 5.05 | 5.09 | 5.06 | 4.86 | 5.65 | |

### Example 7: Mycophenolate mofetil production from Mycophenolic acid, according to WO 02/100855.

100 g of crude mycophenolic acid, prepared according the example 5 were put in a reaction flask with a reflux cooler together with 200 ml dibutyl ether. Under continual stirring the mixture was warmed up to temperature of 50 to 60°C and then 40 ml 2-morpholinoethanol were added. The reaction mixture was warmed up to boiling under azeotropic separation of water. After 48 hours the mixture was cooled up to 23°C and 200 ml dichloromethane was added. The solution was extracted twice with 100 ml 0.5 M aqueous K₂CO₃ and once with 100 ml of water. Then dichloromethane was distilled off under vacuum and the suspension was cooled up to 10 to 15°C. In this matter synthetized crystalline mycophenolate mofetil was recrystallized from ethyl acetate. After drying the crystals 110 g mycophenolate mofetil was obtained with purity more than 99.0% (HPLC).

## Claims

1. A method of regulating the production of an acid metabolite produced by a microbial cell, comprising regulating the oxygen saturation of a culture medium comprising the microbial cell, wherein the microbial cell is a single strain of *Penicillium* having the accession number of CCM 8364 and the acid metabolite is mycophenolic acid (MPA).

2. The method according to claim 1, comprising providing acid metabolite producing microbial cells in a submerged fermentation culture system and producing the acid metabolite from the microbial cells in such culture, wherein the oxygen saturation of the culture medium is regulated to control pH of the culture medium.

3. The method according to claim 2, wherein the mycophenolic acid is produced in the culture medium to a final concentration of MPA (g/l of culture medium) that is in excess of 1g/l.

4. The method according to any preceding claim, wherein the culture medium comprises a basic nitrogen source and a complex nitrogen source.

5. The method according to claim 4, wherein the complex nitrogen source is selected from hydrolyzed casein, corn steep or mixtures thereof.

6. The method according to claim 4 or claim 5, wherein the basic nitrogen source comprises an amino acid supplement,

7. The method according to claim 6, wherein the amino acid supplement is one or more of glycine, methionine and asparagine.

8. The method according to any preceding claim, wherein the level of oxygen saturation is controlled in the range 5 to 40%.

9. The method according to any preceding claim, wherein the level of oxygen saturation is controlled throughout the entire production phase.

10. The method according to claim 9, wherein the level of oxygen saturation in the culture medium is controlled during a time period selected from the time period from 0 to 24 hours, the time period from 0 to 48 hours, from 24 to 48 hours, and from 48 hours of inoculation.

11. The method according to claim 9 or claim 10, wherein the level of oxygen saturation is controlled until the pH rises due to carbon source depletion at the end of the production phase.

12. The method according to any preceding claim, wherein the culture medium is maintained at a pH greater than pH 4.

13. The method according to claim 12, wherein the pH is maintained by regulating the level of oxygen saturation in the culture medium during a production stage for the acid metabolite.

14. The method according to claim 13, wherein the pH is between about pH 4.5 and about pH 5.5 during the production stage.

15. A method according to any preceding claim wherein, the mycophenolic acid is converted to mycophenolate mofetil.

16. The method according to claim 15, wherein the mycophenolic acid is converted to mycophenolate mofetil by reaction with 2-morpholinoethanol.

17. A strain of *Penicillium sp*. deposited as accession number CCM 8364 at Czech Collection of Microorganisms (CCM) at Masaryk University, Bmo, Czech Republic.

18. The strain according to claim 17 in the form of individual cells, cell aggregates, hyphas, filaments and its aggregates in or on any surface or environment.

19. The strain of *Pencillium sp*. according to claim 17 or claim 18, wherein the strain is capable to produce a final concentration of MPA (g/l of culture medium) that is in excess of 1 g/l.

20. A method for producing an acid metabolite comprising fermenting the strain of any of claims 17 to 19, wherein the acid metabolite is mycophenolic acid (MPA).

21. Use of a strain according to any of claims 17 to 19 in a method according to any of claims 1 to 16.

## Patentansprüche

1. Verfahren zum Regulieren der Produktion eines von einer mikrobiellen Zelle produzierten Säuremetaboliten, bei dem man die Sauerstoffsättigung eines die mikrobielle Zelle umfassenden Kulturmediums reguliert, wobei die mikrobielle Zelle ein einzelner Stamm von *Penicillium* mit der Zugangsnummer CCM 8364 Ist, und wobei der Säuremetabolit Mycophenolsäure (MPA) ist.

2. Verfahren nach Anspruch 1, bei dem man die den Säuremetaboliten produzierenden mikrobiellen Zellen in einem Submersfermentationskultursystem bereitstellt und den Säuremetaboliten von den mikrobiellen Zellen in einer solchen Kultur produziert, wobei die Sauerstoffsättigung des Kulturmediums reguliert wird, um den pH des Kulturmediums zu kontrollieren.

3. Verfahren nach Anspruch 2, wobei die Mycophenolsäure in dem Kulturmedium bis zu einer Endkonzentration von MPA (g/l Kulturmedium) erzeugt wird, die größer als 1 g/l ist.

4. Verfahren nach einem der vorangehenden Ansprüche, wobei das Kulturmedium eine basische Stickstoffquelle und eine komplexe Stickstoffquelle umfasst.

5. Verfahren nach Anspruch 4, wobei die komplexe Stickstoffquelle ausgewählt ist unter hydrolysiertem Casein, Comsteep und Gemischen davon.

6. Verfahren nach Anspruch 4 oder Anspruch 5, wobei die basische Stickstoffquelle eine Aminosäureergänzung umfasst.

7. Verfahren nach Anspruch 6, wobei die Aminosäureergänzung eine oder mehrere unter Glycin, Methionin und Asparagin ist

8. Verfahren nach einem der vorangehenden Ansprüche, wobei das Niveau der Sauerstoffsättigung in dem Bereich von 5 bis 40 % kontrolliert wird.

9. Verfahren nach einem der vorangehenden Ansprüche, wobei das Niveau der Sauerstoffsättigung über die gesamte Produktionsphase kontrolliert wird.

10. Verfahren nach Anspruch 9, wobei das Niveau der Sauerstoffsättigung in dem Kulturmedium kontrolliert wird während eines Zeitbereichs, der gewählt ist aus der Zeitperiode von 0 bis 24 Stunden, der Zeitperiode von 0 bis 48 Stunden, von 24 bis 48 Stunden und von 48 Stunden der Inokulation.

11. Verfahren nach Anspruch 9 oder Anspruch 10, wobei das Niveau der Sauerstoffsättigung kontrolliert wird bis der pH infolge der Abreicherung der Kohlenstoffquelle am Ende der Produktiansphase ansteigt.

12. Verfahren nach einem der vorangehenden Ansprüche, wobei das Kulturmedium bei einem pH-Wert von größer als pH 4 gehalten wird.

13. Verfahren nach Anspruch 12, wobei der pH aufrecht erhalten wird durch Regulieren des Niveaus der Sauerstoffsättigung in dem Kulturmedium während einer Produktionsstufe für den Säuremetaboliten.

14. Verfahren nach Anspruch 13, wobei der pH-Wert zwischen etwa pH 4,5 und etwa pH 5,5 während der Produktionsstufe beträgt.

15. Verfahren nach einem der vorangehenden Ansprüche, wobei die Mycophenolsäure in Mycophenolatmofetil umgewandelt wird.

16. Verfahren nach Anspruch 15, wobei die Mycophenolsäure durch Umsetzung mit 2-Morpholinoethanol in Mycophenolatmofetil umgewandelt wird.

17. Stamm von *Penicillium sp*., hinterlegt mit der Zugangsnummer CCM 8364 bei der tschechischen Sammlung von Mikroorganismen (CCM) an der Masaryk Universität, Brno. Tschechische Republik.

18. Stamm nach Anspruch 17 in der Form von individuellen Zellen, Zellaggregaten, Hyphen, Filamenten und dessen Aggregaten in oder auf einer beliebigen Oberfläche oder einer beliebigen Umgebung.

19. Stamm von *Penicillium sp*. nach Anspruch 17 oder Anspruch 18, wobei der Stamm dazu in der Lage ist, eine Endkonzentration von MPA (g/l Kulturmedium) zu produzieren, die größer ist als 1 g/l.

20. Verfahren zum Produzieren eines Säuremetaboliten, bei dem man den Stamm nach einem der Ansprüche 17 bis 19 fermentiert, wobei der Säuremetabolit Mycophenolsäure (MPA) ist.

21. Verwendung eines Stammes nach einem der Ansprüche 17 bis 19 in einem Verfahren nach einem der Ansprüche 1 bis 16.

## Revendications

1. Procédé de régulation de la production d'un métabolite acide produit par une cellule microbienne, comprenant la régulation de la saturation d'oxygène d'un milieu de culture comprenant la cellule microbienne, dans lequel la cellule microbienne est une souche unique de *Penicillium* ayant le numéro d'accession de CCM 8364 et le métabolite acide est l'acide mycophénolique (MPA).

2. Procédé selon la revendication 1, comprenant la fourniture d'un métabolite acide produisant des cellules microbiennes dans un système de culture de fermentation submergé et la production du métabolite acide à partir de cellules microbiennes dans une telle culture, dans lequel la saturation d'oxygène du milieu de culture est régulée pour contrôler le pH du milieu de culture.

3. Procédé selon la revendication 2, dans lequel l'acide mycophénolique est produit dans le milieu de culture à une concentration finale de MPA (g/l de milieu de culture) qui est en excès de 1 g/l.

4. Procédé selon l'une quelconque des revendications précédentes, dans lequel le milieu de culture comprend une source d'azote basique et une source d'azote complexe.

5. Procédé selon la revendication 4, dans lequel la source d'azote complexe est choisie à partir de caséine hydrolysée, d'extrait de maïs ou de mélanges de ceux-ci.

6. Procédé selon les revendications 4 ou 5, dans lequel la source d'azote basique comprend un supplément d'acide aminé.

7. Procédé selon la revendication 6, dans lequel le supplément d'acide aminé est un ou plusieurs glycine, méthionine et asparagine.

8. Procédé selon l'une quelconque des revendications précédentes, dans lequel le niveau de saturation d'oxygène est contrôlé dans la gamme de 5 à 40%.

9. Procédé selon l'une quelconque des revendications précédentes, dans lequel le niveau de saturation d'oxygène est contrôlé pendant toute la phase de production.

10. Procédé selon la revendication 9, dans lequel le niveau de saturation d'oxygène dans le milieu de culture est contrôlé pendant un laps de temps choisi parmi le laps de temps de 0 à 24 heures, le laps de temps de 0 à 48 heures, de 24 à 48 heures, et à partir de 48 heures d'inoculation.

11. Procédé selon la revendication 9 ou la revendication 10, dans lequel le niveau de saturation d'oxygène est contrôlé jusqu'à ce que le pH augmente du fait de l'épuisement de la source de carbone à la fin de la phase de production.

12. Procédé selon l'une quelconque des revendications précédentes, dans lequel le milieu de culture est maintenu à un pH supérieur au pH 4.

13. Procédé selon la revendication 12, dans lequel le pH est maintenu en régulant le niveau de saturation d'oxygène dans le milieu de culture pendant une phase de production pour le métabolite acide.

14. Procédé selon la revendication 13, dans lequel le pH est compris entre environ le pH 4,5 et environ le pH 5,5 pendant la phase de production.

15. Procédé selon l'une quelconque des revendications précédentes, dans lequel l'acide mycophénolique est converti en mofétil de mycophénolate.

16. Procédé selon la revendication 15, dans lequel l'acide mycophénolique est converti en mofétil de mycophénolate par réaction avec du 2-morpholinoéthanol.

17. Souche de *Penicillum sp*. déposée sous le numéro d'accession CCM 8364 à la Czech Collection of Microorganisms (CCM) à l'Université Masaryk, Brno, République Tchèque.

18. Souche selon la revendication 17 sous la forme de cellules individuelles, d' agrégats cellulaires, d'hyphes, de filaments et ses agrégats dans ou sur n'importe quelle surface ou environnement.

19. Souche de *Penicillum sp*., selon la revendication 17 ou la revendication 18, dans laquelle la souche est capable de produire une concentration finale de MPA (g/l de milieu de culture) qui est en excès de 1 g/l.

20. Procédé de production d'un métabolite acide comprenant la fermentation de la souche selon l'une quelconque des revendications 17 à 19, dans lequel le métabolite acide est l'acide mycophénolique (MPA).

21. Utilisation d'une souche selon l'une quelconque des revendications 17 à 19 dans un procédé selon l'une quelconque des revendications 1 à 16.
